# EUROPEAN PATENT APPLICATION

(11) **EP 2 348 127 A1**
(43) Date of publication of application: **27.07.2011**
(21) Application number: 10000732.7
(22) Date of filing: 25.01.2010
(51) Int. Cl.: C12Q 1/18, C12Q 1/68, G01N 21/64

(54) **Method for producing a biosensor for an in vitro screening system for identifying anti-infective substances, and uses thereof**

(71) Applicant: Albert-Ludwigs-Universität Freiburg, 79085 Freiburg (DE)
(72) Inventor: Bumann, Dirk, 4125 Riehen (CH); Mecklenbräuker, Ingrid, 79194 Gundelfingen (DE); Schleberger, Christian, 79106 Freiburg (DE); Rubner, Yvonne, 79104 Freiburg (DE); Hahn, Christiane, 79100 Freiburg (DE)
(74) Representative: Krauss, Jan

(57) **Abstract**

The present invention relates to a method for producing a biosensor for *an in vitro* screening system for identifying anti-infective substances, comprising the steps of a) cloning random genomic DNA fragments from at least one organism of interest upstream of a promoter-less reporter gene into a vector, in order to generate a random genomic library, b) transforming said random genomic library into a host strain, c) selecting clones with anti-infective-responsive reporter expression from said host strains, comprising treating said clones with either i) at least one known anti-infective substance, or ii) at least one potentially anti-infective substance, and iii) comparing said treated clones from i) or ii) with at least one untreated control sample, and d) identifying those clones exhibiting a different anti-infective-responsive reporter expression as biosensors. The invention furthermore relates to *an in vitro* screening system for identifying anti-infective substances as produced according to the invention, preferably comprising at least two complimentary biosensor clones. The invention also relates to uses of said screening system, in particular for high-throughput screening (HTS) in order to identify molecules which inhibit bacterial growth, and can be used as antimicrobially effective substances.

## Description

The present invention relates to a method for producing a biosensor for an *in vitro* screening system for identifying anti-infective substances, comprising the steps of a) cloning random genomic DNA fragments from at least one organism of interest upstream of a promoter-less reporter gene into a vector, in order to generate a random genomic library, b) transforming said random genomic library into a host strain, c) selecting clones with anti-infective-responsive reporter expression from said host strains, comprising treating said clones with either i) at least one known anti-infective substance, or ii) at least one potentially anti-infective substance, and iii) comparing said treated clones from i) or ii) with at least one untreated control sample, and d) identifying those clones exhibiting a different anti-infective-responsive reporter expression as biosensors. The invention furthermore relates to an *in vitro* screening system for identifying anti-infective substances as produced according to the invention, preferably comprising at least two complimentary biosensor clones. The invention also relates to uses of said screening system, in particular for high-throughput screening (HTS) in order to identify molecules which inhibit bacterial growth, and can be used as antimicrobially effective substances.

### Background of the invention

Antibiotics are an essential part of modern medicine. The emergence of antibioticresistant mutants among bacteria is seemingly inevitable, and results, within a few decades, in decreased efficacy and withdrawal of the antibiotic from widespread usage. The traditional answer to this problem has been to introduce new antibiotics that kill the resistant mutants. Unfortunately, after more than 50 years of success, the pharmaceutical industry is now producing too few antibiotics, particularly against Gram-negative organisms, to replace antibiotics that are no longer effective for many types of infection.

As an example, Coates and Hu (in Coates AR, Hu Y. Novel approaches to developing new antibiotics for bacterial infections. Br J Pharmacol. 2007 Dec;152(8):1147-54. Epub 2007 Aug 20) review possible new ways to discover novel antibiotics. They describe that the genomics route has proven to be target rich, but has not led to the introduction of a marketed antibiotic as yet. Non-culturable bacteria may be an alternative source of new antibiotics. Bacteriophages have been shown to be antibacterial in animals, and may find use in specific infectious diseases. Developing new antibiotics that target non-multiplying bacteria is another approach that may lead to drugs that reduce the emergence of antibiotic resistance and increase patient compliance by shortening the duration of antibiotic therapy. These new discovery routes have given rise to compounds that are in preclinical development, but, with one exception, have not yet entered clinical trials.

For the time being, the majority of new antibiotics that reach the marketplace are likely to be structural analogues of existing families of antibiotics or new compounds, both natural and non-natural which are screened in a conventional way against live multiplying bacteria.

In case of antibacterial agents that are to be identified from compound libraries or biological extracts, three different main approaches are currently used.
1. Simple growth assays test the ability of a compound to inhibit bacterial growth. These rapid and easy assays have yielded a number of highly useful compound classes. On the other hand, such screenings have been increasingly less efficient, since many antibacterial compounds that are identified act through unspecific modes of action (e.g. damage of membranes) that likely affect also human cells, bringing with them a high risk of adverse reactions when used for therapy. Resource-consuming follow-up is required to identify a small minority of potentially useful compounds identified in the primary screen.
2. Biochemical assays with purified target enzymes can yield inhibitors with high potency for a defined target and with a well characterized mode of action. The vast majority of these identified compounds do not penetrate the cell envelope of bacterial and other pathogens and/or compounds that enter the cell are rapidly removed by bacterial efflux pumps. As a consequence, these compounds cannot sufficiently inhibit the target enzyme to destroy or otherwise effectively inactivate the pathogens, such as bacteria. Such inhibitors are furthermore extremely difficult to develop into compounds with activity and safety against live bacteria.
3. Furthermore, so-called biosensors in living bacteria responding to perturbations resulting in inhibition of bacterial growth through well characterized modes of action were used to detect antibacterial compounds. In contrast to biochemical assays as mentioned above, this strategy also provides evidence that the inhibitor was able to contact/penetrate its target in the intact bacteria or other pathogen. Other than the simple growth assays, properly designed biosensors can selectively respond to defined antibacterial and anti-infective modes of action, and can distinguish these from certain unspecific toxic damage.

Urban et al (in: Urban A, Eckermann S, Fast B, Metzger S, Gehling M, Ziegelbauer K, Rübsamen-Waigmann H, Freiberg C. Novel whole-cell antibiotic biosensors for compound discovery. Appl Environ Microbiol. 2007 Oct;73(20):6436-43. Epub 2007 Aug 24) describe cells containing reporters which are specifically induced via selected promoters and their use in pharmaceutical drug discovery and in environmental biology. The cells are used as biosensors in screenings for novel drug candidates and in the detection of bioactive compounds in environmental samples. Generated and validated were a set of five *Bacillus subtilis* promoters fused to the firefly luciferase reporter gene suitable for cell-based screening, enabling the as yet most-comprehensive high-throughput diagnosis of antibiotic interference in the major biosynthetic pathways of bacteria: a) the biosynthesis of DNA by the yorB promoter, b) of RNA by the yvgS promoter, c) of proteins by the yheI promoter, d) of the cell wall by the ypuA promoter, and e) of fatty acids by the fabHB promoter.

Yagi (in Yagi K. Applications of whole-cell bacterial sensors in biotechnology and environmental science. Appl Microbiol Biotechnol. 2007 Jan;73(6):1251-8. Epub 2006 Nov 17) describes certain biosensors that have major advantages over chemical or physical analyses with regard to specificity, sensitivity, and portability. Whole-cell bacterial biosensors have been developed using recombinant DNA technology. The bacteria are genetically engineered to respond to the presence of chemicals or physiological stresses by synthesizing a reporter protein, such as luciferase, beta-galactosidase, or green fluorescent protein. Since bacteria possess a wide variety of stress-response mechanisms, including antioxidation, heat-shock responses, nutrient-starvation, and membrane-damage responses, DNA response elements for several stress-response proteins can be fused with various reporter genes to construct a versatile set of bacterial biosensors for a variety of analytes. Cell array biosensors have been designed for high-throughput analysis. Signals from sensors can be detected with digital imaging, epifluorescence microscopy, and/or flow cytometry.

Even the best reported antibacterial biosensors still respond to certain stress conditions and, therefore, detect both potentially interesting specifically acting antibacterial compounds and undesired unspecific inhibitors. As a result, resource-consuming follow up characterization is again required to identify a minority of potentially useful antibacterial compounds out of the primary screen.

Increasing resistance of major human bacterial pathogens to currently used antimicrobials generates an urgent need for new antibacterial agents. In order to quickly and effectively identify new and promising compounds that may serve as such antimicrobial and/or anti-infective agents, improved biosensor systems have to be developed.

The present invention thus in a first aspect thereof provides a method for producing a biosensor for *an in vitro* screening system for identifying anti-infective substances, comprising the steps of a) cloning random genomic DNA fragments from at least one organism of interest upstream of a promoter-less reporter gene into a vector, in order to generate a random genomic library, b) transforming said random genomic library into a host strain, c) selecting clones with anti-infective-responsive reporter expression from said host strains, comprising treating said clones with either i) at least one known anti-infective substance, or ii) at least one potentially anti-infective substance, and iii) comparing said treated clones from i) or ii) with at least one untreated control sample, and d) identifying those clones exhibiting a different anti-infective-responsive reporter expression as biosensors.

Thus, according to the present invention, in a first step of the method random genomic DNA fragments from at least one organism of interest are cloned upstream of a promoter-less reporter gene into a vector, and thus a random genomic library is generated.

Random genomic fragments can be generated by cleaving the genomic DNA, which may be purified and isolated or largely purified and isolated, chemically, physically (e.g. by ultrasound or other mechanical force), and/or enzymatically (e.g. by enzymatic digestion through endonucleases).

Organisms of interest are those organisms, against which an anti-effective substance shall be developed (or against organisms related thereto), and can include any potentially pathogenic organism, in particular a bacterial, fungal, viral or protozoal pathogen, such as pathogenic bacteria that cause infectious diseases, preferably selected from Mycobacterium, Streptococcus, Enterococcus, Neisseria, Pseudomonas, Shigella, Campylobacter, Salmonella, Helicobacter pylori, Staphylococcus, Rickettsia, Chlamydia, Bacillus, Clostridium, and Brucella. Fungal pathogens are, for example, selected from Yeasts, such as Candida. Protozoan pathogens are, for example, Plasmodium, Amoeba, Cryptosporidium, and Giardia. Viral pathogens are, for example, viruses selected from Adenoviridae, Herpesviridae, Hepadnaviridae, Papillomaviridae, and Parvoviridae.

As a promoter-less reporter gene, any gene can be used whose expression can be suitably detected. The detection can take place either directly (e.g. by measuring mRNA or protein expression or indirectly (through detecting fluorescence). Examples for preferred reporter genes are luciferase, beta-galactosidase, or green fluorescent protein (GFP) and are also described further below.

Any plasmid or other vector that is suitable in order to generate a random genomic library in a desired host strain can be used as a vector. Preferably, a suitable vector, such as a host-strain specific vector or a shuttle vector carries sites for an expedited cloning of DNA fragments and one or more selection markers, such as antibiotic resistance markers. One or even more promoter-less reporter gene(s) (or suitable part(s) thereof) is/are cloned into said vector, in order to be potentially expressed by a DNA fragment cloned in front (i.e. in a position to induce expression of said reporter gene in said host strain) of said reporter gene.

At the end of the first step of the inventive method, a random genomic library comprising a multitude of vectors harboring a reporter gene together with a DNA fragment cloned in front of said reporter gene is generated. Optimally, said genomic library includes sufficient DNA fragments in order to encompass all or at least a significant part of the genome of the organism of interest cloned into said vectors. Nevertheless, selected parts of the DNA of a genome can be cloned into said library, e.g. by choosing a particular restriction enzyme that generates longer or shorter fragments of the genome (e.g. depending from the frequency of cutting). Also, a particular range of fragment sizes can be cloned into the vector (e.g. fragments that are likely to cover preferably only the promoter-sequences of said organism that is responsible for the induction of the expression of said reporter-gene), such as fragments of between 50-1000, preferably 200-800, and even more preferred between 500-700 bases in length.

Then, in a second step of the method according to the present invention, said random genomic library is transformed into a host strain. Methods for transforming hosts strains (in particular bacterial host strains) are well known in the state of the art and include chemical (e.g. CaCl-mediated transformation) and physical (e.g. electroporation-mediated) methods.

As a host, any suitable organism for harboring and the expression of the vectors of the library can be used. Preferably, the host organism is selected from a well-established non-pathogenic bacterial strain, such as E. coli or B. subtilis. Nevertheless, also yeast strains or even strains of mammalian, such as human, cells can be used for the purposes of the present invention. Human cells would be preferred in case of DNA libraries derived from protozoan or viral pathogens.

In the third step of the method according to the present invention clones with a presence or a change in anti-infective-responsive reporter expression are identified and selected from said host strains.

In one alternative of the method, clones of the recombinant host strain (or, e.g. in first round of screening, a pool of clones) are treated (or screened) with at least one known anti-infective substance. Exemplary anti-infective substances are known to the person of skill, and are further described below. In this case, the methods seeks to identify and select DNA-fragments of the organism of interest that are activated (or induced) or inactivated (or shut off) by known anti-infective substances, be it a substance that is known to be effective against the organism of interest in this case or any other organism of interest (as described above), such as, for example substances known to be effective against a group of organisms of interest (in some instances also called broad-band antibiotics), such as Gram-negative or Gram-positive bacteria, or substances known to be effective against viruses or fungal pathogens.

In a second alternative of the method, clones of the recombinant host strain (or, e.g. in first round of screening, a pool of clones) are treated (or screened) with at least one potentially anti-infective substance. Said at least one potentially anti-infective substance can be present in a compound library, such as a commercial synthetic compound library, or in a natural product extract, such as a plant extract or other natural substances containing extract.

Next, in both the treated clones from the alternatives above (or pools in a first round of screening and individualized clones in a subsequent round of screening against the compounds or substances) the presence and/or level of expression of the reporter gene(s) is determined. The methods for such determination depend on the conditions of the screening and the kind of reporter gene used, and are known to the person of skill, and also described further below. Examples involve quantitative PCR, antibodies (labeled or unlabeled) for protein detection, enzymatically mediated detectable reactions and/or fluorescence determination.

The results of the determinations as above are then compared to at least one untreated control value derived from at least one control sample, which usually is a value obtained from a clone (or pool of clones) of the host cell as screened that is not treated with said compound or substance as above. As a further or alternative control, a clone of the host cell only carrying the promoter-less reporter gene in a vector without a DNA-fragment and/or a host cell without the vector can be used as an additional control, in order to avoid cloned DNA-fragment-independent false positive or false negative results. The comparison to untreated control cells can preferably involve transcriptomics/proteomics techniques such as DNA microarrays, deep sequencing of reverse transcribed cDNA, two-dimensional gel electrophoresis of proteins, nanoLC-mass spectrometry of tryptic peptides.

Finally, in the fourth step of the method according to the present invention clones exhibiting a different anti-infective-responsive reporter expression are identified and can be selected as biosensors in the context of the present invention. In further preferred embodiments of the method according to the present invention, the biosensors as identified as herein can be further characterized as described below.

In summary, biosensors according to the present invention can therefore be obtained through a variety of methods according to the present invention, namely
(i) using direct cloning of known antibacterial-responsive regulatory DNA-sequences from organisms of interest upstream of a reporter gene such as GFP, luciferase, beta-galactosidase, into host strains, or
(ii) using selection of antibacterial-responsive regulatory sequences from a random promoter-trap library, optimally yielding superior biosensors compared to (i), as it further allows a comprehensive optimization of regulatory sequences under conditions relevant to screening (plasmid properties, growth conditions, assay conditions, strain selection, etc.).

Further embodiments of the method according to the present invention comprise a mutagenesis of biosensors as produced according to the present invention, e.g. as obtained as mentioned in (i) or (ii) above, in order to optimize regulatory sequences or regulatory factors that influence biosensor response patterns. Said mutagenesis can be performed either by introducing mutations into the genome of the host strain (modified background), or, preferably, by introducing mutations into the vector as transformed into the host strain, in particular into the DNA-fragment of the organism of interest as cloned into said vector. Methods for mutagenesis, such as for the introduction of point mutations, either random or specific, are known to the person of skill, and are also further described below, such as site-directed mutagenesis of regulatory DNA sequences, e.g. through modifying or deleting the spacing between the -35 and -10 boxes of the promoter, generating translational fusions, overexpression of proteins, or relieving the post-transcriptional control by inserting a stop codon.

In view of the above, preferred is a method for producing a biosensor for an *in vitro* screening system for identifying anti-infective substances according to the present invention, wherein the random genomic DNA fragments are produced through shearing and/or restriction digestion of DNA.

Further preferred is a method for producing a biosensor for *an in vitro* screening system for identifying anti-infective substances according to the present invention, wherein the at least one organism of interest is selected from fungi, protozoans, parasites, viruses and bacteria, such as pathogenic bacteria, in particular human or animal pathogenic bacteria, Gram-positive and Gram-negative bacteria.

Also preferred is a method for producing a biosensor for an *in vitro* screening system for identifying anti-infective substances according to the present invention, wherein said reporter gene is selected from GFP, destabilized GFP variants such as GFP_OVA, YFP, GFPuv, luciferase, and beta-galactosidase.

Still more preferred is a method for producing a biosensor for *an in vitro* screening system for identifying anti-infective substances according to the present invention, wherein said vector is selected from promoter trap vectors, such as pFB1, pFB2, pFB3, or pFB4.

Even more preferred is a method for producing a biosensor for *an in vitro* screening system for identifying anti-infective substances according to the present invention, wherein said host strain is selected from non-pathogenic and/or pathogenic bacteria, human pathogenic bacteria, Gram-positive bacteria, and Gram-negative bacteria, such as *E. coli* or *B. subtilis.*

Also preferred is a method for producing a biosensor for an *in vitro* screening system for identifying anti-infective substances according to the present invention, wherein said at least one known anti-infective substance is selected from ampicillin, kanamycin, evernimicin, fluoroquinolones, bacitracin, cefotaxim, cefsulodin, cycloserine, fosfomycin, imipenem, vancomycin, cerulenin, thiolactomycin, triclosan (Irgasan), avilamycin, azithromycin, blasticidin, chloramphenicol, puromycin, gentamycin, hygromycin, neomycin, streptomycin, tetracycline, actinomycin, ciprofloxacin, novobiocin, phleomycin, rifampicin, and polymyxin.

Still preferred is a method for producing a biosensor for an *in vitro* screening system for identifying anti-infective substances according to the present invention, wherein said at least one potentially anti-infective substance is included in a compound library, such as a commercial synthetic compound library, or in a natural product extract. Respective libraries and methods for using them in preferably high-throughput-screening (HTS) are known to the person of skill and described in the literature.

Yet another preferred aspect of the present invention then relates to a method for producing a biosensor for an *in vitro* screening system for identifying anti-infective substances according to the present invention which further comprises determining a) the specificity and sensitivity of said reporter gene expression in response to at least one known anti-infective substance having a defined mode of anti-infective action, and/or b) the reporter gene expression in response to at least one cellular stress condition. This provides for the further characterization of the biosensors and thus leads to the ability to design more specific *in vitro* screening systems for identifying anti-infective substances according to the present invention. Preferred is a method for producing a biosensor for an *in vitro* screening system for identifying anti-infective substances according to the present invention, wherein said analyzing of the specificity and sensitivity of said reporter gene expression comprises analyzing the maximal induction of GFP expression in the presence of the reference antibiotics, the standard deviation, and/or the screening performance Z'-score. Further preferred is a method for producing a biosensor for an *in vitro* screening system for identifying anti-infective substances according to the present invention, wherein said defined mode of anti-infective action and/or cellular stress condition is selected from inhibiting bacterial functions that are essential *in vitro* and *in vivo* during infection, such as cell wall and protein biosynthesis, peptidoglycan biosynthesis, protein biosynthesis, fatty acid biosynthesis, DNA replication, RNA polymerase, division functions; DNA replication/transcription/intercalation, disruption of the cell membrane, cellular lysis, chelating agents, oxidizers, solvents, protein denaturation, antioxidation, heat-shock responses, nutrient-starvation, and membrane-damage.

Yet another preferred method for producing a biosensor for *an in vitro* screening system for identifying anti-infective substances according to the present invention further comprises optimizing the specificity and sensitivity of said biosensor through site-directed mutagenesis of regulatory DNA sequences, such as, by modifying or deleting the spacing between the -35 and -10 boxes of the promoter, generating translational fusions, overexpression of proteins, or relieving the post-transcriptional control by inserting a stop codon.

In another important aspect of the method for producing a biosensor for an *in vitro* screening system for identifying anti-infective substances according to the present invention, said method further comprises combining at least two or more different vectors for screening as above in one host cell. Further preferred is a combination of three, or even four, vectors comprising biosensors as identified.

Another preferred aspect of the present invention then relates to a biosensor produced according to a method according to a method of the present invention as described herein.

In the context of the present invention, the term "biosensor" shall mean a biological system, and preferably a host cell as described herein, that comprises at least one of the vectors of the library as described above, and is suitable for expressing said vector(s) in order to allow for a screening according to the present invention. Preferably, a biosensor according to the present invention is a bacterial host strain as described herein that is transfected with at least one of the vectors of the library as described above.

According to the present invention, preferred cellular biosensors can include various bacterial species such as Gram-positive, Gram-negative, and other species. Since these biosensors are live bacteria, only compounds that can penetrate the cell envelope and can maintain sufficient internal concentrations even in the presence of active efflux pumps will have an effect on the expression and can be detected. This avoids a main disadvantage of the common target-based screening according to the state of the art.

A further preferred aspect of the present invention thus also relates to new recombinant bacterial strains as biosensors having an altered sensitivity to defined antibacterial substances, produced according to the method according to the present invention.

Yet another aspect of the present invention then relates to *an in vitro* screening system for identifying anti-infective substances, comprising a biosensor as produced according to the present invention as described herein.

Preferably, *the in vitro* screening system for identifying anti-infective substances according to the present invention comprises at least two or more, preferably complimentary, biosensors as identified. Further preferred are three, or even four, preferably complimentary, biosensors as identified. In the context of the present invention, complimentary biosensors are biosensors relating to different mode of actions of the (known) antibacterial substance, such inhibiting bacterial functions that are essential *in vitro* and *in vivo* during infection, such as cell wall and protein biosynthesis, peptidoglycan biosynthesis, protein biosynthesis, fatty acid biosynthesis, DNA replication, RNA polymerase, and cell division functions. The biosensors can be combined on a plate in different wells for, for example, HTS screening, but also can be combined in one multiplex assay sample as a mixture of biosensors, either as single cellular sensors and/or cells containing several sensors.

Preferred is thus a method for producing the *in vitro* screening system for identifying anti-infective substances according to the present invention, wherein at least two different biosensors are combined, and wherein first a potential biosensor, such as a newly identified biosensor according to the present invention, or a known biosensor that shall be characterized further are tested against a set of inhibitors and/or stress conditions as described further herein, and thus a biosensor-specific pattern is generated and/or extended regarding their modes of action response, and, optionally, a preliminary toxic profile is generated. Preferably, then a biosensor is considered further that exhibits a largely uniform reaction against inhibitors and/or stress conditions, and does not respond to an essential extent to at least one other inhibitor and/or stress condition. This "specific" biosensor is then preferably combined with at least one other biosensor showing a largely uniform reaction against inhibitors and/or stress conditions different from the first biosensor. These biosensors are regarded as "complementary" in the context of the present invention.

The person of skill will be able to select different groups of modes-of-action based on the description herein and the literature as published in the field. As a further preferred example, the following known antibacterial substances could be used in order to analyze and/or characterize modes of action for biosensors to be identified/used in the context of the present invention.

**Table 1: Antibacterial substances to be used in order to analyze and/or characterize modes of action for biosensors**

| Mode-of action | Antibacterial substance |
|---|---|
| Inhibiting cell wall biosynthesis | Bacitracin, Cefotaxim, Cefsulodin, Cycloserin, Fosfomycin, Imipenem, Vancomycin |
| Inhibiting cell wall biosynthesis/fatty acid biosynthesis | Cerulenin, Thiolactomycin, Triclosan (Irgasan) |
| Inhibiting protein biosynthesis (at the 50 S subunit) | Avilamycin, Azithromycin, Blasticidin S, Chloramphenicol, Puromycin |
| Inhibiting protein biosynthesis (at the 30 S subunit) | Gentamycin, Hygromycin B, Kanamycin, Neomycin, Streptomycin, Tetracyclin |
| DNA replication/transcription/intercalation | Actinomycin, Ciprofloxacin, Novobiocin, Phleomycin, Rifampicin |
| Unspecific disruptors of the cell membrane | Polymyxin, Polymyxin B sulfate |
| Unspecific cellular lysis compounds | SDS |
| Unspecific chelating agents | EDTA |
| Unspecific oxidizing agents | Hydrogen peroxide |
| Unspecific solvents | DMSO, ethanol |
| Unspecific protein denaturants | Urea |
| Unspecific other stress conditions | Potassium thiocyanate (KSCN), Sodium chloride (NaCl), Triton-X-100 |

Further preferred is therefore an *in vitro* screening system for identifying anti-infective substances according to the present invention, wherein the combination of biosensors is specifically selected for identifying cell wall biosynthesis inhibitors, fatty acid biosynthesis inhibitors, RNA polymerase inhibitors, gyrase inhibitors, topoisomerase inhibitors, protein biosynthesis inhibitors, protein biosynthesis inhibitors that bind to the ribosome at a site close to the avilamycin binding-site, or antibacterial compounds active against Gram-negative and/or Gram-positive bacterial pathogens, Mycobacteria, Treponema, and/or Mollicutes.

Other preferred examples for combinations are described in the examples and the accompanying figures, and show the advantageous enhanced specificity of, for example, peptidoglycan inhibitor detection using biosensor combinations according to the invention. Peptidoglycan biosynthesis inhibitors show a response pattern distinct from all other stress conditions including cell envelope stress caused by polymyxin. Further, the specific detection of peptidoglycan biosynthesis or protein biosynthesis inhibitors using *Bacillus subtilis* biosensor combinations was shown. The present invention even allows for an identification of novel antimicrobial compounds acting through a yet unexploited mode of action using biosensors with differential resistance against the protein biosynthesis inhibitor avilamycin.

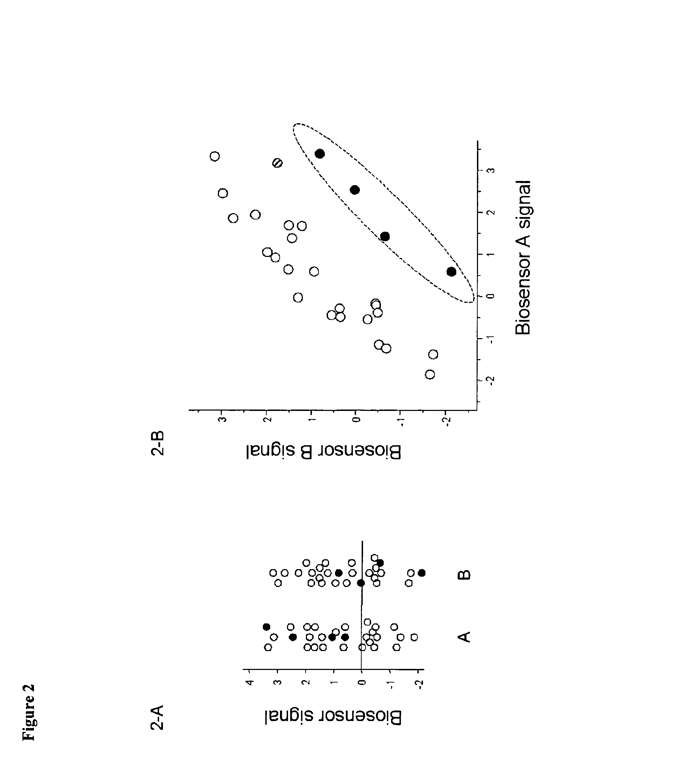

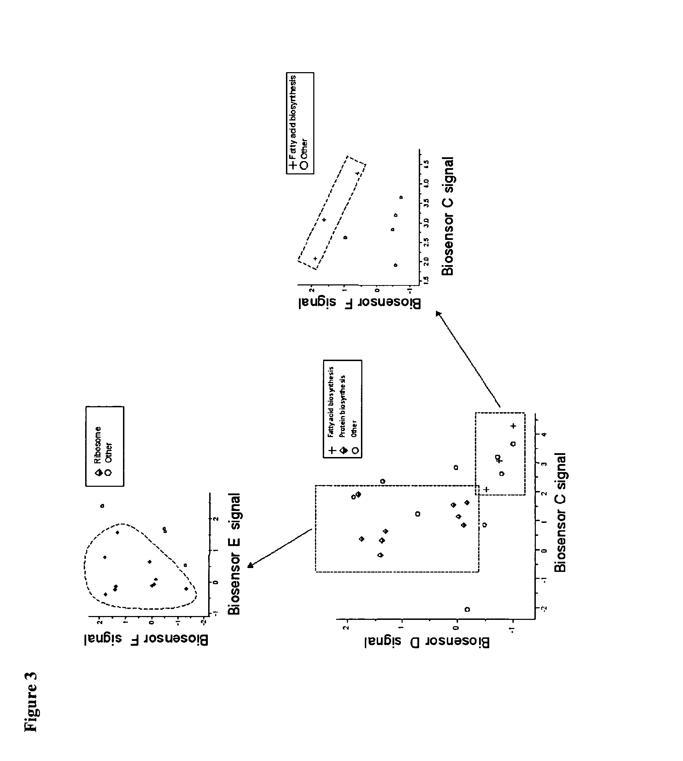

As an example to illustrate how the specificity is enhanced following the method described in this invention using a system featuring two biosensors, biosensors A and B can be used. Biosensor A responds to peptidoglycan biosynthesis inhibitors, but also to other stress conditions as indicated in FIG. 2A. On the other hand, biosensor B shows weak responses to peptidoglycan damage (FIG. 2B). However, a comparison of the responses of biosensors A and B revealed that this combination identifies peptidoglycan inhibitors with very high specificity. Furthermore, polymyxin that targets the cell envelope through a different mode of action, induced a response pattern clearly distinct from those of peptidoglycan damage. The results shows superior specificity for detection of antimicrobials with defined mode of action. The antibiotics and other stress conditions with biosensor A/B response patterns distinct from peptidoglycan damage can be further preferably be separated using further biosensors, biosensor C and D as illustrated in FIG. 3. Biosensor C responds also to peptidoglycan biosynthesis inhibitors, and less to other stress conditions. Biosensor D is generally induced by various protein biosynthesis inhibitors, but repressed by fatty acid biosynthesis inhibitors. To further enhance specificity, compounds with biosensor C/D response patterns typical for protein biosynthesis inhibition can also be analyzed using biosensors E and F as also shown in FIG. 3. This analysis clearly separates protein biosynthesis inhibitors from other stress conditions. Similarly, compounds with biosensor C/D response patterns typical for fatty acid biosynthesis inhibition can also be further analyzed with biosensor F to distinguish substantial fatty acid biosynthesis inhibitors from other stress conditions (FIG. 3). Thus, preferred is a combination of 2 biosensors according to the invention, preferably 3 biosensors according to the invention, and more preferred 4 or 5 biosensors according to the invention, wherein said biosensors each show at least one clearly distinct response pattern for a mode of action and/or stress condition as described herein from each other.

Preferred is an *in vitro* screening system for identifying anti-infective substances according to the present invention that comprises at least one biosensor (clone) comprising an anti-infective-responsive reporter expression identified through transcriptome/proteome-based selection, with the provisio that at least one biosensor clone has not been identified through said transcriptome/proteome-based selection.

Most preferred is the *in vitro* screening system for identifying anti-infective substances according to the present invention, which is suitable for HTS screening, such as in high density well plates, e.g. 1536, 384- or 96-well plates, preferably 96-well plates.

The present invention thus provides a preferred *in vitro* screening system based on a combination of selective and complementary biosensors for identifying compounds that inhibit bacterial growth, and simultaneously for characterizing their mode of action, and, optionally, generating a preliminary toxic profile.

In particular, the system allows for selectively identifying compounds that inhibit microbes, such as pathogenic bacteria through defined mode-of-actions, but excludes compounds acting through unspecific toxic effects. The system preferably comprises biosensors according to the present invention in living bacteria that respond to defined antibacterial mode-of-actions. In contrast to previous biosensor-based assays that suffer from limited specificity, the current system involves a combination of complementary biosensors that are monitored simultaneously.

The system has the following advantages compared to prior screening approaches:
(i) Selective identification and/or characterization of anti-bacterial substances with useful mode-of-action already in the primary screen,
(ii) Enhanced and/or more specific responses through combination of complimentary biosensors,
(iii) Simultaneous identification of compounds that penetrate into organisms of interest, such as live bacteria, and
(iv) Rapid testing even at low compound concentrations.

Yet another important aspect of the present invention then relates to an *in vitro* method for screening anti-infective substances comprising contacting at least one potentially anti-infective substance with the *in vitro* screening system according to the present invention, and identifying the potentially anti-infective substance based on a difference in the anti-infective-responsive reporter expression, compared to a control, in analogy as described above for the biosensor screening. Methods for screening are known to the person of skill and can include color or fluorometric assays, enzymatic reactions, and binding assays, mainly in dependence from the marker or reporter gene to be detected. Preferred is an *in vitro* method for screening anti-infective substances according to the present invention, wherein said at least one potentially anti-infective substance is comprised in a compound library, such as a commercial synthetic compound library, or in a natural product extract.

Further preferred is an *in vitro* method for screening anti-infective substances according to the present invention, wherein a mixture of individual biosensors is combined as described above, and their responses are measured in parallel, for example in an HTS screening format, and/or using high density well plates, e.g. 384-or 96-well plates, preferably 96-well plates. Respective technology and assays are known to the person of skill and further described herein.

Particularly preferred is an *in vitro* method for screening anti-infective substances according to the present invention, wherein multiple fluorescent proteins are detected in parallel using flow cytometry, fluorescence plate readers, and/or bioluminescence plate readers. In addition, in a also preferred *in vitro* method for screening anti-infective substances according to the present invention, additional parameters, such as biosensor cell number, biosensor cell size, biosensor membrane properties, are assayed and/or recorded.

In view of the above, the present invention thus generally provides a preferred *in vitro* method based on a combination of selective and complementary biosensors according to the invention, (i) to identify compounds that inhibit bacterial growth, and simultaneously; (ii) to characterize the mode of action of said compounds, and (iii) to describe a preliminary toxic profile.

In particular, this method according to the invention can selectively identify compounds that inhibit bacteria or other pathogenic microbes through defined mode-of-actions, and allows to exclude compounds acting through unspecific toxic effects. The method according to the invention utilizes biosensors in living bacteria that respond to defined antibacterial mode-of-actions. In contrast to previous biosensor-based assays that suffer from limited specificity, the current method preferably involves a combination of complementary biosensors that are monitored simultaneously. The thus detected entire response profile provides uniquely specific and sensitive data on bacterial and/or microbial inhibition and its mode-of-action. Added preferred benefits can be a short incubation time of 2 to 8 hours, preferably 2-5 hours in high density well plates, e.g. 386- or 96-well plates, preferably 96-well plates and detectable response levels at low sub-lethal compound concentrations (up to 100-fold below the minimal inhibitory concentration). This also enables identification of hits (effective compounds) having a low water solubility.

The invented method has the following advantages compared to prior screening approaches:
(i) Selective identification of anti-bacterial substances with useful mode-of-action already in the primary screen,
(ii) Highly specific responses through combination of complimentary biosensors,
(iii) Simultaneous identification of compounds that penetrate into live bacteria, and
(iv) A rapid assay at low compound concentrations.

Mode-of-actions of interest according to the present invention are preferentially chosen to cover bacterial functions that are essential *in vitro* and *in vivo* during infection, such as peptidoglycan biosynthesis, protein biosynthesis, fatty acid biosynthesis, DNA replication, RNA polymerase, cell division functions. Additional mode-of-actions can be added as needed. Biosensor responses preferably can be measured automatically with appropriate equipment such as fluorescence/bioluminescence plate readers or automated flow cytometers. Flow cytometry is a particularly useful technique since up to 20 parameters can be accurately quantified on the single biosensor level.

Another aspect of the present invention then relates to a method for generating a response profile for the anti-infective substance screened according to the present invention as above, comprising combining the response data from several different biosensors. In contrast to previous biosensor-based assays that suffer from limited specificity, the current method preferably involves a combination of complementary biosensors that are monitored simultaneously. The thus detected entire response profile provides uniquely specific and sensitive data on bacterial and/or microbial inhibition and its mode-of-action.

Since the response pattern is specific for defined mode-of-action, antibacterial compounds with non-attractive mode-of-action are excluded already as a result of the primary screen. Expensive follow-up of useless compounds is thus minimized. This avoids a main disadvantage of simple growth assay-based screening according to the state of the art.

In another preferred embodiment of the method according to the present invention, the method further comprises comparing and/or combining said response profile as generated with a response profile for a set of antibacterial molecules with known modes of action. This additional information can be used to further improve selection of effective response profiles as a tool for the development of effective treatments and related strategies.

Another important aspect of the present invention relates to a method for treating a bacterial infection, comprising the method for screening an anti-infective substance according to the present invention, and administering an effective amount of said anti-infective substance to a patient in need of said treatment. Effective anti-infective treatment regimens are known to the person of skill and the attending physicians, and can be readily adjusted to the patients' needs. Furthermore, combination therapies are included in the scope of the present invention.

Preferred is a method for treating a bacterial infection according to the present invention, wherein said anti-infective substance or a combination of anti-infective substances is administered based, at least in part, on the response profile for said anti-infective substance or the response profiles for said anti-infective substances as generated according to the invention as described herein.

Another important aspect of the present invention then relates to the use of the *in vitro* screening system according to the present invention for screening anti-infective substances, in analogy to what is described above.

Preferably, for biosensor construction the present invention utilizes a novel combination of biosensors to detect chemical compounds that inhibit bacterial growth through a defined mode of action. To obtain such biosensors, in general regulatory DNA sequences that respond to defined antibacterial mode of actions are fused to reporter genes. These constructs are then transformed into bacterial host cells.

For the selection of regulatory DNA sequences, suitable regulatory DNA sequences that respond to model antimicrobial compounds with well defined mode of action are preferably obtained by two independent approaches:
(i) Transcriptome/proteome-based selection of regulatory DNA sequences has been the method of choice in the prior-art (see e.g. Urban et al. Novel whole-cell antibiotic biosensors for compound discovery in AEM 73: 6463-6443 (2007) and references therein).

In this approach, a) bacterial cells are treated with sublethal concentrations of model compounds for various periods of time, b) mRNA and/or proteins are extracted from these cells and compared to untreated control cells using transcriptomics/proteomics techniques such as DNA microarrays, deep sequencing of reverse transcribed cDNA, two-dimensional gel electrophoresis of proteins, nanoLC-mass spectrometry of tryptic peptides, or complementary methods. Genes with different expression levels in antimicrobial-treated vs. untreated bacterial cells are called antimicrobial-responsive genes. Associated potential regulatory sequences (promoters, transcription factor binding sites, ribosomal binding sites, sRNA binding sites, etc.) are identified through analysis of genomic context (operon structure, intergenic regions, etc.) of antimicrobial-responsive genes. Candidate regulatory DNA sequences are fused to reporter genes such as luciferase, beta-galactosidase, green fluorescent protein variants or other fluorescent proteins, etc., and the constructs are transformed into bacterial host cells. The resulting biosensor candidates are tested for antimicrobial-responsive reporter gene expression. Confirmed biosensor candidates are evaluated for specificity and sensitivity of reporter gene expression using a set of antimicrobials and of the stress conditions with different modes of antibacterial action.

Transcriptomics/proteomics-based selection of regulatory DNA sequences is usually successful in only a subset of cases. Discrepancies between response patterns of regulatory sequences in their native genomic context vs. reporter gene fusions can be caused by differences in gene copy number, DNA topology, context, etc. Such problems can be avoided by an alternative approach based on random genomic libraries which is the most preferred method of choice.
(ii) Selection of regulatory sequences from random genomic libraries. In such libraries, each regulatory sequence can be tested at the same copy number, topology, and context as in the final biosensor candidate.

In this method, bacterial genomic DNA is isolated and sheared into fragments of 500 to 700 bp length using ultrasonication as previously described in Bumann and Valdivia. Identification of host-induced pathogen genes by differential fluorescence induction reporter systems in Nature Protocols 2: 770-777 (2007). Fragments are cloned upstream of a promoter-less reporter gene. The resulting library is transformed into bacterial cells by known methods.

Clones with antimicrobial-responsive reporter expression are selected from the library by comparing treated and untreated control clones (or pools thereof). This can also be done by picking individual clones and automated testing in multi-well plates. Alternatively, libraries with reported genes encoding fluorescent proteins can be sorted for antimicrobial-responsive clones using flow cytometry as previously described in Bumann and Valdivia. Identification of host-induced pathogen genes by differential fluorescence induction reporter systems in Nature Protocols 2: 770-777 (2007).

Antimicrobial-responsive clones represent biosensor candidates that are subsequently evaluated for specificity and sensitivity of reporter gene expression using a set of antimicrobials and of the stress conditions with different modes of antibacterial action.

For a biosensor modification according to another preferred aspect according to the present invention, the specificity and sensitivity of biosensor candidates is preferably further optimized by two novel complementary approaches.

### (i) Site-directed mutagenesis of regulatory DNA sequences.

In this approach transcription factor binding sites can be modified or deleted as follows:
a) The spacing between the -35 and -10 boxes of the promoter can be changed by insertion or deletion of base pairs.
b) Translational fusions can be generated by in-frame connecting N-terminal fragments of genes encoded in the 3' part of the regulatory DNA sequence with downstream reported genes. This enables biosensor modulation by post-transcriptional regulation.
c) Alternatively, post-transcriptional control can be relieved by insertion of a stop codon.

### (ii) Modification of signaling inputs.

Regulatory DNA sequences of integrate inputs from several signaling pathways that respond to a variety of stimuli. Simplification of signaling inputs can improve the specificity for a single defined mode of antibacterial action. This can be achieved by inactivating certain signaling pathways through gene deletion of key factors. Potentially relevant signaling pathways are identified based on experimentally verified or predicted transcription factor binding sites, or based on gene expression data for the locus of interest in wild type bacteria vs. signaling pathway mutants.

Regarding reporter genes and readout methods, a variety of reporter genes has been previously used to measure biosensor responses. In particular, luciferase generates bioluminescence that can be measured with high sensitivity and low background. Beta-galactosidase can be used to convert colorless/non-fluorescent substrates into colored/fluorescent products.

What has not been used previously for this purpose, and is the preferred choice according to the present invention, is the use of fluorescent proteins as reporter genes that do not require any additional processing step. Indeed, fluorescent proteins which are intrinsically less sensitive reporters compared to luciferase and beta-galactosidase offer other distinct advantages. For instance, multiple fluorescent proteins can be detected in parallel, thus enabling simple and efficient multiplexing. Moreover, fluorescent proteins can be easily detected and measured on the single cell level using flow cytometry which enables to selectively monitor responsive subpopulations in complex heterogeneous mixtures.

Preferred according to the present invention are multiplex assay, since all previously reported biosensors for antimicrobial mode of actions show some lack of specificity when comprehensively evaluated under diverse stress conditions. Positive biosensor responses might thus indicate antibacterial compounds with the desired mode of action, but also compounds with undesired unspecific toxicity. Resource-consuming secondary screens are thus required to distinguish between these two alternatives. In contrast to these inefficient biosensor assays, the present invention preferably utilizes a combination of biosensors with different functions in a single assay simultaneously to overcome the above mentioned drawback. Each biosensor still has limited specificity when analyzed alone. However, comparative analysis of the differential response patterns from several biosensors acting in parallel generates a profile that is highly specific for a particular antibacterial compounds' mode of action.

As an example, one particular biosensor, biosensor A, responds strongly to specific cell wall biosynthesis inhibitors but weakly responds to unspecific membrane-damaging compounds. When used alone, biosensor A would thus detect both desired and undesired antibacterial compounds. On the other hand, another biosensor, biosensor B, responds to both, cell wall biosynthesis inhibitors and membrane damaging agents, but in contrast to A it shows a stronger signal for membrane damaging agents. When detecting the signals from biosensor A (preferentially responding to cell-wall inhibitors), and biosensor B (preferentially responding to membrane damaging agents) simultaneously, desired cell wall biosynthesis inhibitors (rendering signal A > signal B) can be clearly distinguished from membrane damaging agents (rendering signal A < signal B). As a result, comprehensive measurement and analysis of signals from biosensor combinations yields superior selectivity with detailed information on antibacterial mode of actions already in the primary screen. Resource-consuming follow-up studies can, therefore, be restricted to a few highly promising hit compounds with desired mode of action, as described further below.

In order to obtain response profiles of suitable biosensor combinations, the response profile of each biosensor is first determined individually. Subsequently, the results are combined for comprehensive analysis. For this approach according to the invention, the same or different reporter genes can be used in each biosensor.

Alternatively, the individual biosensors can be combined in a mixture in one well and their responses measured in parallel. This has the advantage of identical conditions for all biosensors, smaller amounts of compounds required for testing, and fewer pipetting steps and, it takes into account any possible cross interaction from the individual biosensors. In this case, each biosensor must be individually labeled to distinguish its response from those of the other biosensors in the mixture. Suitable options include differential fluorescence labeling using spectrally different variants of the green fluorescent protein. Alternatively, biosensors can also be labeled using reactive fluorescent dyes for covalent coupling or, use luciferase variants emitting at different wavelengths.

Flow cytometry is a particularly useful readout system for multiplex biosensors and is the preferred choice according to the present invention, since biosensor cells are measured at single cell resolution which enables their efficient separations according to different responses. An interesting feature of biosensor cell counting for the present purpose is the use fluorescent beads as an internal calibration standard that provides accurate, sensitive, and rapid information on the net antibacterial activity of compounds.

Moreover, flow cytometers with simultaneous detection of multiple fluorescence and scatter properties enable recording of additional parameters such as biosensor cell number, biosensor cell size, biosensor membrane properties, etc. These additional parameters provide complementary information that further increases response specificity and sensitivity. Combined signals from several different biosensors yield response profiles that contain information on potential antibacterial modes of action of the tested compound. Profiles are evaluated by comparison to response profiles for a large set of antibacterial molecules with well-characterized modes of action.

Another important aspect of the present invention then relates to a method for generating an antibiotic pattern for a particular organism of interest (or information on the effectiveness of anti-infective substances against said organism of interest), using an in vitro screening system according to the present invention.

Data analysis from multiplexed flow cytometers can be easily automated for processing and interpretation using various commercially available software packages such as 'R' and similar packages.

Another important aspect of the present invention thus relates to a method for generating an antibiotic database for a particular organism of interest or a group of organisms of interest (or information on the effectiveness of anti-infective substances against said organism of interest), using an in vitro screening system according to the present invention.

Additional objects, advantages and features of this invention will become apparent to those skilled in the art upon reading the following examples, which are not intended to be limiting the scope of the invention. In the figures,
Figure 1 shows the detection of antimicrobial biosensor responses using fluorescent reporter proteins and flow cytometry. A) Histogram showing the GFP fluorescence intensities of an E. coli clone responsive to the model antibiotic cefotaxime. B) Dot plots demonstrating multi-color separation of E. coli biosensors expressing GFP, YFP, or GFPuv, respectively. Shown are fluorescence intensities of selected E. coli biosensors.
Figure 2 shows the enhanced specificity of peptidoglycan inhibitor detection using biosensor combinations according to the invention. A) Individual biosensor signals (log₂(fluorescence induction)) of biosensors A (in wildtype E. coli K-12) and B (in E. coli K-12 rcsF) to a wide variety of model antibiotics and other stress conditions at half maximal bacterial growth. Peptidoglycan biosynthesis inhibitors (cycloserine, fosfomycin, cefsulodine, imipineme) are represented by filled circles. B) Comparison of biosensors A and B responses to the same compounds. Peptidoglycan biosynthesis inhibitors show a response pattern distinct from all other stress conditions including cell envelope stress caused by polymyxin (hatched).
Figure 3 shows the specific detection of protein biosynthesis or fatty acid biosynthesis inhibitors using additional E. coli biosensor combinations. This analysis clearly separates protein biosynthesis inhibitors from other stress conditions.
Figure 4 shows the specific detection of peptidoglycan biosynthesis or protein biosynthesis inhibitors using *Bacillus subtilis* biosensor combinations.
Figure 5 shows the specific identification of novel antimicrobial compounds acting through a yet unexploited mode of action using biosensors with differential resistance against the protein biosynthesis inhibitor avilamycin.
Figure 6 shows the competitive growth of *Bacillus* wildtype, *Bacillus* avi^{sp}, and *Bacillus* avi^{meth} in the presence of various natural product extracts.

SEQ ID No. 1 and 2 show primers fb1 and fb2 as used in example 1, below.

### EXAMPLES

### EXAMPLE 1: Combination of Escherichia coli biosensors for antimicrobial HTS

According to the present invention, in order to obtain biosensors that can detect antimicrobials acting through defined modes of action in an unbiased manner, large random promoter-trap libraries were generated as illustrated in the examples described below. These libraries were subsequently screened for clones that show increased reporter gene expression when treated with well-characterized antibiotics with defined modes of action. The resulting biosensors exhibited superior specificity for detection of antimicrobials with defined mode of actions.

### Construction of promoter-trap libraries

A set of different promoter libraries was made by inserting random *E. coli* K12 DNA fragments (500-1000 bp) into promoter trap vectors, creating gene fusions to a promoter-less gfp protein, and transformed into *E. coli* K12 strain MG1655.

The promoter trap plasmids pFB1 and pFB2, are medium copy plasmids derived from pMW82 (Gen Bank accession number EF363313), a pBR322-(colE1 bla) vector carrying a promoter-less unstable *gfp* variant (gfp_ova; pFB1) or stable *gfp* (pFB2), a transcriptional terminator (rrnB) and an ampicillin resistance (AmpR) cassette as previously described in Rollenhagen et al. (2004). Antigen selection based on expression levels during infection facilitates vaccine development for an intracellular pathogen in PNAS 101:8739-8744. Stable GFP can accumulate to high levels and is thus optimal for detecting weak promoters. In case of strong promoters, however, stable GFP can reach toxic levels that could impair biosensor viability. To detect such strong promoters, destabilized GFP variants such as GFP_OVA are more suitable as previously described (Bumann and Valdivia (2007). Identification of host-induced pathogen genes by differential fluorescence induction reporter systems. Nature Protocols 2: 770-777). To cover both strong and weak promoters, two independent libraries using either GFP variant were constructed.

The two stop codons immediately in front of the *gfp* / *gfp_ova* gene were removed by site-directed mutagenesis, so that a translational read-through of genes on the genomic insert into downstream *gfp* was possible. In this way, both transcriptional and translational regulation can be utilized for optimal biosensor performance.

Vectors pFB1 and pFB2 were cut with BamHI and blunted using T4 DNA polymerase. Chromosomal DNA from *E. coli* K12 MG1655 was isolated using a standard procedure (Current Protocols in Molecular Biology (2001) John Wiley & Sons, eds.) to generate predominantly DNA fragments in the 500-1000 bp size range. DNA fragments in this size range were used for ligations into pFB1 or pFB2, respectively.

Electroporation of the ligation mixture into *E. coli* XL10 (Stratagene) yielded pFB1 and pFB2 independent clones, 100 % of which carried an insert. The colonies from all plates were pooled and the residual bacterial suspension was used to prepare plasmid DNA using a standard procedure (Current Protocols in Molecular Biology (2001) John Wiley & Sons, eds.). These plasmid preparations were then electroporated into the original source of the genomic inserts, *E. coli* K12 MG1655, yielding 1.5 (stable gfp) or 2.2 (unstable gfp) x 10⁶ clones that represent a >50-fold coverage of the *E. coli* K12 MG1655 genome based on insert size, dual orientations, genome size and the three different open reading frames. The colonies from all plates were again pooled and resuspended in sterile L-broth/7% (vol/vol) DMSO. Aliquots of this promoter-trap library were then frozen and stored at -80 °C.

### Screening of promoter-trap libraries for antimicrobial biosensors

To enrich potential biosensor candidates, the promoter trap libraries for clones with active promoter-*gfp* fusions were screened in the presence of one model antibiotic out of a set of cell wall and protein biosynthesis inhibitors (e.g. Cefotaxime, Cycloserine, Fosfomycin, Polymyxin, Chloramphenicol, Kanamycin and Tetracyclin). In a second step, these clones were tested for differential GFP expression in presence and absence of the model antibiotic.

Aliquots of the promoter trap libraries were resuspended in *Spizizen's* minimal medium as described (Stewart (1968). Enhancement and inhibition of transformation of Bacillus subtilis in J Bact. 95: 2428-2430). One model antibiotic was added at a concentration that inhibits bacterial growth by 50%, followed by a sufficient incubation period and conditions.

5'000 to 10'000 bacteria expressing GFP above background levels under these conditions were individually sorted into 96-well plates containing-L-broth using a flow cytometer. GFP-positive bacteria were detected based on scatter properties (forward and sideward scatter) and green (502-535 nm) fluorescence emission after excitation with a 488 nm laser. A green fluorescence threshold that excluded 99.99% of GFP-negative control bacteria, was used. Plates containing individual sorted bacteria were incubated and stored.

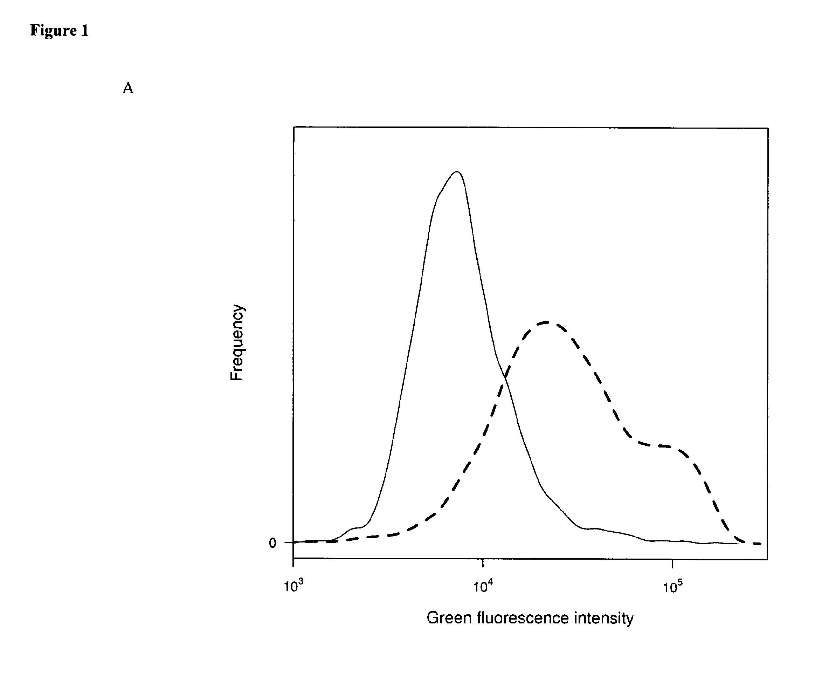

Aliquots of each sorted clone were analyzed for GFP expression in the presence or absence of the model antibiotic that was used for the initial sorting (FIG. 1-A). Most clones showed GFP expression in both conditions (constitutively active promoter fusions), while some clones showed antibiotic-dependent GFP expression (regulated promoter fusions). Clones of the second class were additionally tested for responses to a wide variety of antibiotics and other stress conditions to identify clones with suitable selective antibiotic response patterns. The genomic inserts of these biosensor candidates were PCR-amplified with flanking primers "fb1" 5'-GTGATGTCGGCGATATAG (SEQ ID No. 1) and "fb2" 5'-GAATTGGGACAACTCCAG (SEQ ID No. 2), followed by restriction mapping with AluI, HpaII and Tsp905I restriction endonucleases to identify unique inserts. Unique inserts were sequenced and compared with available DNA sequence databases (Gen Bank (NCBI), Colibri (complete dataset of DNA sequences of *E. coli* K12) using nblast homology searches.

For each potential biosensor strain, the maximal induction of GFP expression in the presence of the reference antibiotics, the standard deviation and the screening performance Z'-score that measures signal-to-background separation and predicts false-positive hit rates were determined using a known method (Zhang et al. (1999) A simple statistical parameter for use in evaluation and validation of high throughput screening assays in J Biomol Screen 4: 67-73); the criteria used was: 1 > Z' > 0.9 is assumed an excellent assay, 0.9 > Z' > 0.7 a good assay while 0.7 > Z' > 0.5 an useful assay. Assay conditions including bacterial inoculation, bacterial density, growth conditions, and analysis parameters were optimized to maximize the Z'-score. Only biosensors with Z'-scores above 0.7 were selected for further development. All experiments were performed in the presence of DMSO.

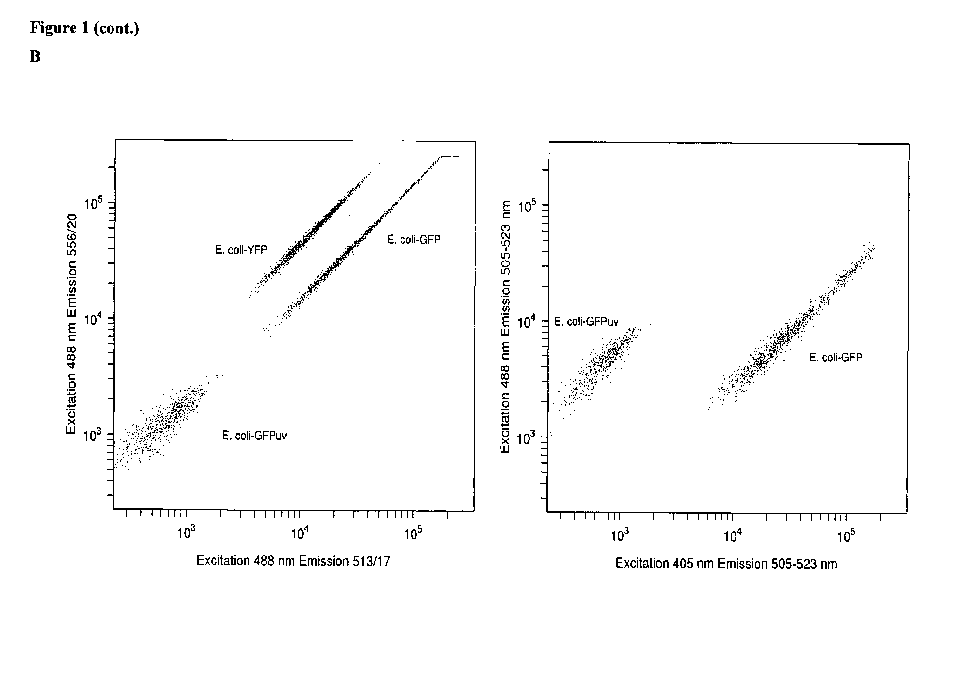

In the present invention, the comparison of simultaneous responses from multiple biosensors render detailed information on antimicrobial modes of action. To maximize the throughput parallel measurement and the various biosensors is beneficial. To enable such multiplex analysis of our biosensors, the reporter genes in selected biosensors from GFP is exchanged for other fluorescent proteins that can be distinguished from each other and GFP using flow cytometry. Typical results are shown in **FIG. 1****B.**

### Specific detection of antimicrobials with defined mode of action

Each biosensor obtained as explained above was responsive to certain model antibiotics but also showed some unspecific responses to antibiotics with unrelated modes of action and/or other stress conditions. This imperfect specificity is a result of complex signaling networks in the bacterium that integrate multiple stimuli into a broad response pattern. Similar problems have previously been reported for other antimicrobial biosensors (e.g. Urban et al. (2007). Novel whole-cell antibiotic biosensors for compound discovery in AEM 73: 6463-6443).

As an example to illustrate how the specificity is enhanced following the method described in this invention using a system featuring two biosensors, biosensors A and B were used. Biosensor A responds to peptidoglycan biosynthesis inhibitors, but also to other stress conditions as indicated in FIG. 2A. On the other hand, biosensor B shows weak responses to peptidoglycan damage (FIG. 2B). However, a comparison of the responses of biosensors A and B revealed that this combination identifies peptidoglycan inhibitors with very high specificity. Furthermore, polymyxin that targets the cell envelope through a different mode of action, induces a response pattern clearly distinct from those of peptidoglycan damage. Separation by cell wall stress caused by peptidoglycan damage vs. polymyxin has not been reported for any antimicrobial biosensor and the results shows superior specificity for detection of antimicrobials with defined mode of action.

Antibiotics and other stress conditions with biosensor A/B response patterns distinct from peptidoglycan damage can be further separated using further biosensors, biosensor C and D as illustrated in FIG. 3. Biosensor C responds also to peptidoglycan biosynthesis inhibitors, and less to other stress conditions. Biosensor D is generally induced by various protein biosynthesis inhibitors, but repressed by fatty acid biosynthesis inhibitors.

To enhance specificity, compounds with biosensor C/D response patterns typical for protein biosynthesis inhibition were also analyzed using biosensors E and F as also shown in FIG. 3. This analysis clearly separates protein biosynthesis inhibitors from other stress conditions. Similarly, compounds with biosensor C/D response patterns typical for fatty acid biosynthesis inhibition can also be further analyzed with biosensor F to distinguish substantial fatty acid biosynthesis inhibitors from other stress conditions (FIG. 3).

### Screening of compound libraries using biosensor combinations

A library of 52,000 commercial synthetic compounds was screened using the novel biosensors mentioned in the previous section using 96-well plates and a flow cytometer (LSRII with HTS-loader, BD Biosciences) using fluorescent beads (Crimson Beads, Invitrogen) as an internal standard. As the biosensors are live bacteria, this provides additional information on antimicrobial activity of each compound. Forward and side scatter as well as four additional (GFP, YFP, GFPuv, FM4-64) fluorescence parameters were simultaneously detected. The data analysis was carried out by automatic batch analysis with a dedicated computer software. Raw data was processed with scripts written in the statistical programming language R (R Development Core Team (2008) *R: A Language and Environment for Statistical Computing,* R Foundation for Statistical Computing, Vienna, Austria) using libraries from the bioconductor project (Gentleman et al. (2004). Bioconductor: open software development for computational biology and bioinformatics in Genome Biology 5: R80).

78 primary hits (confirmed in independent replicates) were found in the screening. The screening also correctly identified the few well-known antimicrobial compounds Cefoperazone, Sparfloxaxin and Ciprofloxacin that where present in the library and constitute a strong proof-of-concept of the power of the method described in the present invention.

The here described screening assay could also be used to screen natural product extracts. However, complex natural product extracts pose additional challenges for screening because of frequent re-identification of previously characterized antimicrobial activities. To identify novel promising activities these already known activities ("de-replication") need to be discriminated. To overcome this additional difficulty a series of spontaneous *E. coli* K-12 mutants with high resistance against various cell wall biosynthesis inhibitors was generated. Such mutants are subsequently combined with promoter-*gfp* fusions as those described in the present disclosure to generate biosensors that specifically respond to previously unknown cell wall biosynthesis inhibitors.

### EXAMPLE 2: Combination of Bacillus subtilis biosensors for antimicrobial HTS

The procedures described in EXAMPLE 1 were also used to construct two large random promoter-trap libraries from the Gram-positive bacterium *Bacillus subtilis* strain 168. In this example used promoter trap plasmids pFB3 (carrying a reporter gene encoding stable GFP) and pFB4 (carrying a reporter gene encoding destabilized GFP[AAV] were used as previously described (Andersen et al. (1998). New unstable variants of green fluorescent protein for studies of transient gene expression in bacteria in AEM 64: 2240-2246). Both plasmids were derived from pDG148-Stu, a shuttle vector for *E. coli* and *B. subtilis* as previously described (Joseph et al. (2001). Rapid oriented cloning in a shuttle vector allowing modulated gene expression in Bacillus subtilis in FEMS Microbiol. Lett. 205: 91-97). Clones with promising biosensor properties were identified from these libraries using the same strategies as described in EXAMPLE 1 for *E. coli* K-12.

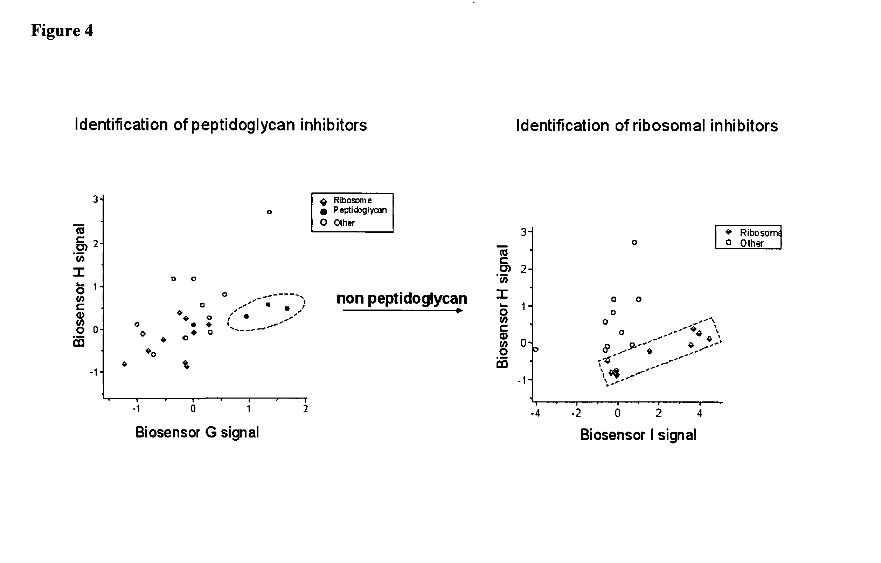

Similar to what was found for *E. coli,* comparison of response patterns of different *B. subtilis* biosensors to the same compounds enabled the identification of antibiotics acting through defined modes of action with high specificity as shown in FIG. 4. Indeed, biosensor G responded strongly to peptidoglycan biosynthesis inhibitors (Cefotaxim, Cycloserin, Vancomycin HCl, but not to Fosfomycin) but also to unrelated stresses such as Ciprofloxacin and H₂O₂. However, a combination of biosensor G with biosensor H clearly separated peptidoglycan biosynthesis inhibitors from other stress conditions as also shown in FIG. 4.

Antibiotics and stress conditions with biosensor G/H response patterns different from peptidoglycan inhibition could be further separated using, for example, biosensor I. Comparison of biosensor H and biosensor I responses revealed a distinct response pattern for protein biosynthesis inhibitors (FIG. 4).

These data demonstrate that biosensor combinations can also be used to identify antimicrobials acting against Gram-positive bacteria through specific modes of action.

**EXAMPLE 3: Combination of *Bacillus subtilis* biosensors to identify novel antibiotics that specifically target one defined binding site on the ribosome**

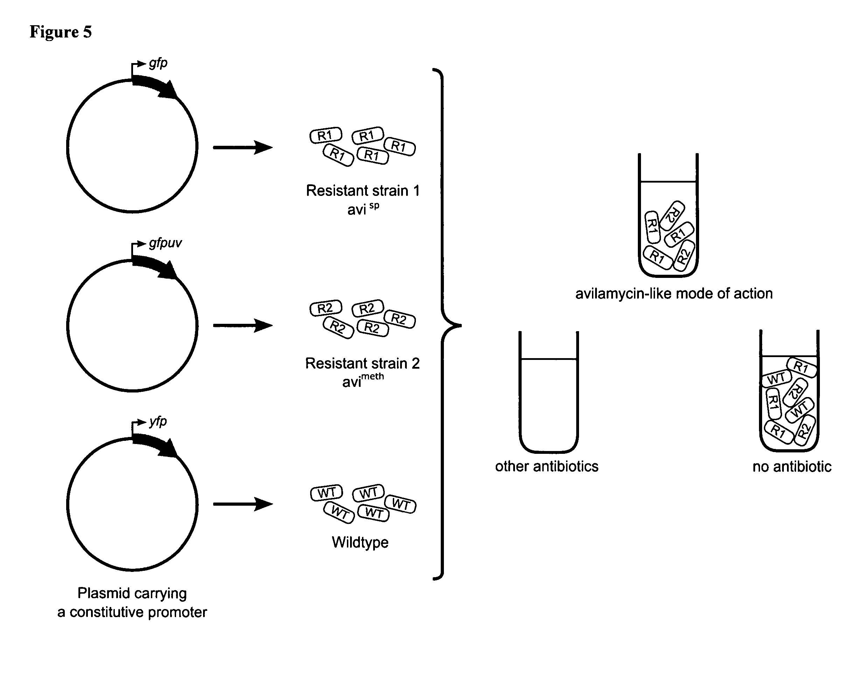

As an alternative approach to generate biosensors for antimicrobial screening, bacterial strains with different resistance to a well-characterized but so far unexploited mode of action can be used. Some novel antimicrobial compounds acting through a specific mode of action would kill a normal sensitive strain but not the resistant strain. In contrast, antimicrobial compounds acting through other modes of action might kill both strains, while inactive compounds would affect neither strain. Based on relative growth of susceptible and resistant strains, it is thus possible to identify novel antimicrobials acting through the desired mode of action as illustrated in FIG. 5.

Specifically, avilamycin is an oligosaccharide antibiotic which is produced by *Streptomyces viridochromogenes* Tü57 and is known to be mainly active against Gram-positive bacteria (Gäumann et al. (1960) *Herstellung und Gewinnung des Antibiotikums Avilamycin.* DE 1116864 and Buzzetti et al. (1968). Avilamycin. Experientia 24: 320-323). Avilamycin has been used as a growth promoter in piglets, pigs, chickens and turkeys for several years in Europe until antibiotic growth promoters were banned. Avilamycin and a related compound, evemimicin, were not allowed in humans for toxicity reasons (Loebenberg et al. (1994). Biological activity of SCH27899 (EVE), an everninomycin antibiotic. 33rd ICAAC, Session 41: Poster 456). Avilamycin is known to inhibit protein biosynthesis by binding to an unique site on the 23S rRNA of the 50S ribosomal subunit but no clinically approved antibiotic targets this same site.

Spontaneous avilamycin-resistant *Bacillus subtilis* strains were obtained by serial passage on media with rising avilamycin concentrations. In total, three independent strains were generated that could grow on media containing up to 100 mg/L avilamycin. Sequencing of the *rplP* gene encoding ribosomal protein L16 revealed single non-synonymous mutations in each strain (amino acid exchanges R51C, I52N, or R56P, respectively). Previous studies found similar L16 mutations causing avilamycin resistance (Aarestrup and Jensen (2000). Presence of Variations in Ribosomal Protein L16 Corresponding to Susceptibility of Enterococci to Oligosaccharides (Avilamycin and Evernimicin). Antimicrob. Agents Chemother. 44: 3425-3427 and Aarestrup and McNicholas (2002). Incidence of high-level evernimicin resistance in Enterococcus faecium among food animals and humans. Antimicrob Agents Chemother. 46:3088-90). None of the mutations were found in region V in any of the 10 *rrl* genes encoding the ribosomal 23S RNA although previous studies showed that such mutations could also contribute to avilamycin resistance (Kofoed and Vester (2002). Interaction of avilamycin with ribosomes and resistance caused by mutations in 23S rRNA. AAC 46: 3339-3346 and Zarazaga et al. (2002). Mutations in ribosomal protein L16 and in 23S rRNA in Enterococcus strains for which evernimicin MICs differ. AAC 46: 3657-3659). The avilamycin-resistant strain *Bacillus avi^{sp}* otherwise showed wild type sensitivity to a large panel of antibiotics with unrelated modes of action as indicated in table 1.

**Table 1. Susceptibility of wildtype B. subtilis and Bacillus avi^{sp} to a wide range of model antibiotics**

| Model antibiotics | Factor Biosensor signal |
|---|---|
| Avilamycin | 65 |
| Actinomycin | 1 |
| Cefotaxim | 1 |
| Cerulenin | 1 |
| Chloramphenicol | 1 |
| Cycloserine | 1 |
| Evemimicin | 4 |
| Gentamycin | 1 |
| Hygromycin | 1 |
| Kanamycin | 1 |
| Neomycin | 1 |
| Phleomycin | 1 |
| Polymyxin | 1 |
| Puromycin | 1 |
| Streptomycin | 1 |
| Tetracyclin | 1 |
| Vancomycin | 1 |

In addition, a *Bacillus* strain was generated that was resistant to avilamycin through an independent mechanism. Specifically, the gene *aviRb* from *S. viridochromogenes* Tü57 (NCBI AF333038) encoding a rRNA methyltransferase methylating the 23S rRNA at helix 91 and helix 89 was cloned downstream of a the constitutive promoter. The AviRb-catalyzed 23S methylation has previously been shown to confer avilamycin resistance (Weitnauer et al. (2001) An ATP-binding cassette transporter and two rRNA methyltransferases are involved in resistance to avilamycin in the producer organism Streptomyces viridochromogenes Tü57. Antimicrob. Agents Chemother. 45: 690-695). Indeed, *Bacillus* transformed with the plasmid carrying the aviRb-casette could grow on media containing up to 100 mg avilamycin l⁻¹. The resulting strain was called *Bacillus avi^{meth}.*

Wildtype *Bacillus* and the two different avilamycin-resistant strains were differentially labeled using plasmid encoded promoter fusions *P-yfp* (wildtype), *P-gfp (Bacillus avi^{sp}),* or P-AviRb-*gfpuv (Bacillus avi^{meth}).*

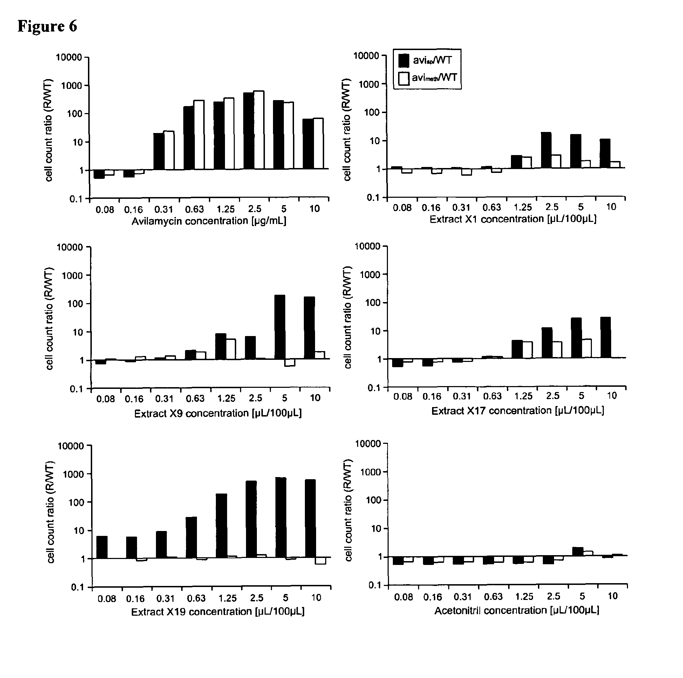

To screen synthetic compounds or natural product extracts, all three fluorescent strains were mixed and distributed onto 96-well plates in which each well contains a different compound (FIG. 5). After competitive growth, fluorescence was measured either using a microplate reader with three filter sets specific for gfp, yfp or gfp-UV or with a flow cytometer. The relative growth yield of each strain in the mixture was determined based on the respective fluorescence signature. Novel compounds with avilamycin-like mode of action diminish YFP, but not GFP or GFPuv fluorescence. Subtle differences in the exact binding mode to the avilamycin site on the ribosome could be revealed by differential GFP vs. GFPuv fluorescence intensities as shown in FIG. 6 for various natural product extracts.

The assay has shown very high specificity as demonstrated by a Z' factor of 0.90 and is suitable for high-throughput screening.

## Claims

1. A method for producing a biosensor for *an in vitro* screening system for identifying anti-infective substances, comprising the steps of
a) cloning random genomic DNA fragments from at least one organism of interest upstream of a promoter-less reporter gene into a vector, in order to generate a random genomic library,
b) transforming said random genomic library into a host strain,
c) selecting clones with anti-infective-responsive reporter expression from said host strains, comprising treating said clones with either
i) at least one known anti-infective substance, or
ii) at least one potentially anti-infective substance, and
iii) comparing said treated clones from i) or ii) with at least one untreated control sample, and
d) identifying those clones exhibiting a different anti-infective-responsive reporter expression as biosensors.

2. The method for producing a biosensor for *an in vitro* screening system for identifying anti-infective substances according to claim 1, wherein said at least one organism of interest is selected from fungi, protozoans, parasites, viruses and bacteria, such as pathogenic bacteria, in particular human or animal pathogenic bacteria, Gram-positive and Gram-negative bacteria.

3. The method for producing a biosensor for *an in vitro* screening system for identifying anti-infective substances according to claim 1 or 2, wherein said at least one known anti-infective substance is selected from ampicillin, kanamycin, evemimicin, fluoroquinolones, bacitracin, cefotaxim, cefsulodin, cycloserine, fosfomycin, imipenem, vancomycin, cerulenin, thiolactomycin, triclosan (Irgasan), avilamycin, azithromycin, blasticidin, chloramphenicol, puromycin, gentamycin, hygromycin, neomycin, streptomycin, tetracycline, actinomycin, ciprofloxacin, novobiocin, phleomycin, rifampicin, and polymyxin.

4. The method for producing a biosensor for an *in vitro* screening system for identifying anti-infective substances according to any of claims 1 to 3, further comprising analyzing
a) the specificity and sensitivity of said reporter gene expression in response to at least one known anti-infective substance having a defined mode of anti-infective action, and/or
b) the reporter gene expression in response to at least one cellular stress condition.

5. The method for producing a biosensor for an *in vitro* screening system for identifying anti-infective substances according to claim 4, wherein said defined mode of anti-infective action and/or cellular stress condition is selected from inhibiting bacterial functions that are essential *in vitro* and *in vivo* during infection, such as cell wall and protein biosynthesis, peptidoglycan biosynthesis, protein biosynthesis, fatty acid biosynthesis, DNA replication, RNA polymerase, division functions; DNA replication/transcription/intercalation, disruption of the cell membrane, cellular lysis, chelating agents, oxidizers, solvents, and protein denaturation.

6. The method for producing a biosensor for an *in vitro* screening system for identifying anti-infective substances according to any of claims 1 to 5, further comprising combining at least two complimentary biosensors as identified.

7. An *in vitro* screening system for identifying anti-infective substances, comprising a biosensor produced according to any of claims 1 to 6.

8. The *in vitro* screening system for identifying anti-infective substances according to claim 7, comprising at least two complimentary biosensor clones produced according to any of claims 1 to 6, and optionally comprising at least one biosensor clone comprising an anti-infective-responsive reporter expression identified through transcriptome/proteome-based selection, with the provisio that at least one biosensor clone has not been identified through said transcriptome/proteome-based selection.

9. The *in vitro* screening system for identifying anti-infective substances according to claim 7 or 8, comprising a mixture of biosensors.

10. The *in vitro* screening system for identifying anti-infective substances according to any of claims 7 to 9, wherein the combination of biosensors is specifically selected for identifying cell wall biosynthesis inhibitors, fatty acid biosynthesis inhibitors, RNA polymerase inhibitors, gyrase inhibitors, topoisomerase inhibitors, protein biosynthesis inhibitors, protein biosynthesis inhibitors that bind to the ribosome at a site close to the avilamycin binding-site, or antibacterial compounds active against Gram-negative and/or Gram-positive bacterial pathogens, Mycobacteria, Treponema, and/or Mollicutes.

11. An *in vitro* method for screening anti-infective substances comprising contacting at least one potentially anti-infective substance with *the in vitro* screening system according to any of claims 7 to 10, and identifying the potentially anti-infective substance based on a difference in the anti-infective-responsive reporter expression, compared to a control.

12. The *in vitro* method for screening anti-infective substances according to claim 11, wherein a mixture of individual biosensors is combined and their responses measured in parallel.

13. The *in vitro* method for screening anti-infective substances according to claim 12, wherein multiple fluorescent proteins are detected in parallel using flow cytometry, fluorescence plate readers, and/or bioluminescence plate readers, and /or wherein additional parameters, such as biosensor cell number, biosensor cell size, biosensor membrane properties, are recorded.

14. A method for generating a response profile for the anti-infective substance screened according to claim 12 or 13 comprising combining the response data from several different biosensors, and optionally further comprising comparing and/or combining said response profile as generated with a response profile for a set of antibacterial molecules with known modes of action.

15. Use of *the in vitro* screening system according to any of claims 7 to 10 for screening anti-infective substances.


<110>  Albert-Ludwigs-Universität Freiburg

<120>  Method for producing a biosensor for an in vitro screening system
        for identifying anti-infective substances, and uses thereof

<130>  FB22716

<160>  2

<170>  PatentIn version 3.5

<210>  1
<211>  18
<212>  DNA
<213>  Escherichia coli

<400>  1
gtgatgtcgg cgatatag                                                    18


<210>  2
<211>  18
<212>  DNA
<213>  Escherichia coli

<400>  2
gaattgggac aactccag                                                    18